# EUROPÄISCHE PATENTANMELDUNG

(11) **EP 1 479 372 A2**
(43) Veröffentlichungstag der Anmeldung: **24.11.2004**
(21) Anmeldenummer: 04012151.9
(22) Anmeldetag: 21.05.2004
(51) Int. Cl.: A61K 7/48, A61K 7/06, A61K 7/50

(54) **Biologische Wirkkomplexe zur Herstellung von Parfümerie- und kosmetischen Erzeugnissen**

(30) Priorität: 21.05.2003 AZ 10103
(71) Anmelder: Safira K. Gamsaeva, Baku 370000 (AZ); Djamila D. Askerova, Baku 370000 (AZ)
(72) Erfinder: Safira K. Gamsaeva, Baku 370000 (AZ); Djamila D. Askerova, Baku 370000 (AZ)
(74) Vertreter: Patentanwälte Zellentin & Partner

(57) **Zusammenfassung**

Die Erfindung betrifft die Kosmetikindustrie, insbesondere biologische Wirkkomplexe auf Basis in der Natur vorkommender Verbindungen natürlichen Ursprungs zur Herstellung von Parfümerie- und kosmetischen Erzeugnissen.

Die erfindungsgemäßen biologischen Wirkkomplexe enthalten zumindest einen biologischen Wirkstoff (BWS) und komplexbildende Bestandteile. In einer Ausführungsform der Erfindungen enthält ein Komplex mit der Bezeichnung "Naphtovit" als BWS 49,3 - 46,2 Gew.-% Naphthalan, und als komplexbildende Bestandteile 5,9 - 6,1 Gew.-% Vitamin E, 7,9 - 8,1 Gew.-% Vitamin C, 5,9 - 6,1 Gew.-% Vitamin F, 0,6 - 0,7 Gew.-% ätherische Öle der Rose, 1,4 - 1,6 Gew.-% ätherische Öle der Geranie, 1,9 - 2,1 Gew.-% ätherische Öle des Basilikums und 26,0-30,0 Gew.-% strukturbildende Bestandteile, bevorzugt Bienenwachs und/oder Lanolin. In einer weiteren Ausführungsform der Erfindung enthält ein Komplex mit der Bezeichnung "Vitavol" als BWS 55,6 - 54,4 Gew.-% vulkanischen Ton, und als komplexbildenden Bestandteil 17,6 -18,4 Gew.-% eines Füllstoffs, bevorzugt Kaolin und/oder Talkum sowie 26,8 - 27,2 Gew.-% niedermolekulare Alkohole, bevorzugt Glycerin und/oder Dipropylenglycol. In einer anderen Ausführungsform der Erfindung enthält ein Komplex mit der Bezeichnung "Vitasu-1" als BWS 89,4 - 67,0 Gew.-% jod- und bromhaltiges Wasser, und als komplexbildende Bestandteile 0,5 - 20,0 Gew.-% Guarkernmehl, 0,1-10,0 Gew.-% Trilon B sowie 10,0 - 30,0 Gew.-% niedermolekulare Alkohole, vorzugsweise Glycerin und/oder Sorbitol und/oder Propylenglycol und/oder Dipropylenglycol.

## Beschreibung

Die Erfindung betrifft die Kosmetikindustrie, insbesondere biologische Wirkkomplexe auf Basis von Ausgangsstoffen natürlichen Ursprungs zur Herstellung von Parfümerie- und kosmetischen Erzeugnissen.

Aus der RU 2183954, Int. KI. A61 K 7/48, 1/30, 02.04.2001, ist ein biologischer Wirkkomplex bekannt, der Bestandteil eines kosmetischen Mittels zur komplexen Hautpflege ist. Dieser Komplex enthält biologisch aktive Wirkstoffe in Form von Extrakten von Heilpflanzen auf wäßriger Glycerinbasis und/oder Liposom-Suspensionen dieser Extrakte, die die Zell- und Systemstrukturen der Haut wirkungsvoll beeinflussen. Dieses kosmetische Mittel kann gesundheitsfördernden Mitteln mit therapeutischer Wirkung zugerechnet werden.

Der Nachteil des bekannten biologischen Wirkkomplexes besteht darin, dass sich seine synergistischen Eigenschaften nur im reinen beschriebenen Komplex zeigen und dieser nicht als Halbfertigerzeugnis zur Herstellung kosmetischer Mittel und Mittel für die Gesundheitspflege eingesetzt werden kann.

Als nächstliegender Stand der Technik wird die RU 2182479, Int. KI. A61 K 7/48, 7/00, 22.05.2001 angesehen, aus der eine Komposition zur Herstellung von Parfümerie- und kosmetischen Erzeugnissen, enthaltend einen biologischen Wirkstoff (BWS) und komplexbildende Bestandteile, bekannt ist. Im bekannten Komplex wird als BWS der alkoholische Extrakt einer Ginseng-Biomasse eingesetzt, und die komplexbildenden Bestandteile umfassen Diethylphthalat oder Essigsäurealdehyd oder Crotonsäurealdehyd oder Diethylether. Die erhaltene Komposition stellt ein komplexes Halbfertigprodukt dar, das als Bestandteil verschiedener kosmetischer Mittel (Lotionen, Cremes, Tonika usw.) sowie als Extrahier- und Lösungsmittel in der Produktion in der Parfümerie- und Kosmetikindustrie verwendbar ist.

Die kosmetischen Mittel, die auf Basis des genannten Komplexes hergestellt werden, tonisieren die Hautzellen, verbessern die Blutversorgung der Haut über die feinen Kapillargefäße und erhöhen ihre Elastizität, was zur Verzögerung des Alterungsprozesses der Haut beiträgt.

Der Nachteil dieses bekannten Komplexes besteht darin, dass die komplexbildenden Bestandteile synthetische Stoffe umfassen, die selbst hautreizend wirken und im Zusammenwirken mit Bestandteilen der Basisprodukte von Erzeugnissen der Parfümerie und Gesundheitspflege beim Anwender Nebenwirkungen wie Hauttrockenheit, Hautreizungen und sogar Allergien hervorrufen können. Außerdem ist die Ginseng-Biomasse, die in diesem Komplex als BWS eingesetzt wird, teuer in der Herstellung.

Die Aufgabe der Erfindung besteht in der Erweiterung des Spektrums biologischer Wirkkomplexe zur Herstellung von kosmetischen Erzeugnissen und Erzeugnissen für die Gesundheitspflege. Diese Aufgabe wird gelöst durch die erfindungsgemäßen neuen biologischen Wirkkomplexe auf Basis von Ausgangsstoffen natürlichen Ursprungs (Naphthalan, vulkanischer Ton, jod- und bromhaltiges Wasser), die mit den Bestandteilen kosmetischer Mittel und Mittel für die Gesundheitspflege kompatibel sind.

Die erfindungsgemäßen biologischen Wirkkomplexe enthalten zumindest einen biologischen Wirkstoff (BWS) und komplexbildende Bestandteile. In einer Ausführungsform der Erfindungen enthält ein Komplex mit der Bezeichnung "Naphtovit" als BWS 49,3 - 46,2 Gew.-% Naphthalan, und als komplexbildende Bestandteile 5,9 - 6,1 Gew.-% Vitamin E, 7,9 - 8,1 Gew.-% Vitamin C, 5,9 - 6,1 Gew.-% Vitamin F, 0,6 - 0,7 Gew.-% ätherische Öle der Rose, 1,4 - 1,6 Gew.-% ätherische Öle der Geranie, 1,9 - 2,1 Gew.-% ätherische Öle des Basilikums und 26,0-30,0 Gew.-% strukturbildende Bestandteile, bevorzugt Bienenwachs und/oder Lanolin. In einer weiteren Ausführungsform der Erfindung enthält ein Komplex mit der Bezeichnung "Vitavol" als BWS 55,6 - 54,4 Gew.-% vulkanischen Ton, und als komplexbildenden Bestandteil 17,6 - 18,4 Gew.-% eines Füllstoffs, bevorzugt Kaolin und/oder Talkum sowie 26,8 - 27,2 Gew.-% niedermolekulare Alkohole, bevorzugt Glycerin und/oder Dipropylenglycol. In einer anderen Ausführungsform der Erfindung enthält ein Komplex mit der Bezeichnung "Vitasu-1" als BWS 89,4 - 67,0 Gew.-% jod- und bromhaltiges Wasser, und als komplexbildende Bestandteile 0,5 - 20,0 Gew.-% Guarkernmehl, 0,1-10,0 Gew.-% Trilon B sowie 10,0 - 30,0 Gew.-% niedermolekulare Alkohole, vorzugsweise Glycerin und/oder Sorbit und/oder Propylenglycol und/oder Dipropylenglycol.

Die Bestandteile der erfindungsgemäßen Komplexe sind, mit Ausnahme des jodund bromhaltigen Wassers, als solche bekannt und deren Verwendung in kosmetischen Mitteln und Mitteln für die Gesundheitspflege allgemein üblich. So beschreibt die SU 1639654, A61 K 7/50, 1991 ein Reinigungsmittel für das Wannenbad und die Haarwäsche, worin Naphthalan als biologischer Wirkzusatz zur Förderung der Schaumbildung und zur Senkung des Verbrauchs an oberflächenaktiven Mitteln eingesetzt wird. In der vorliegenden Erfindung hingegen wird Naphthalan als BWS zur Erhöhung der regenerierenden, tonisierenden und heilenden Wirkung von kosmetischen Erzeugnissen und Erzeugnissen für die Gesundheitspflege eingesetzt, wobei die Zusammensetzung der Komplexe so gewählt ist, dass eine synergistische Wirkung erzielt wird, die darin besteht, daß die biologische und antioxidierende Aktivität des Komplexes erhöht und seine Weiterverarbeitung zu hochwirksamen kosmetischen Erzeugnissen mit einer positiven Wirkung auf die physiologischen Hautfunktionen erlaubt. So verstärken zum Beispiel die Vitamine E und C des Komplexes "Naphtovit" als natürliche Antioxidantien und das Vitamin F, das eine wundheilende Wirkung besitzt, gemeinsam mit dem Naphthalan die antioxidierende und wundheilende Aktivität des Gesamtkomplexes und mindern gleichzeitig die reizende Wirkung des Naphthalans. Die Wechselwirkung der Vitamine E und F mit dem Naphthalan erhöht die Durchlässigkeit der Haut für in den kosmetischen Erzeugnissen und Erzeugnissen für die Gesundheitspflege enthaltenen Nährstoffe. Die ätherischen Öle verstärken nicht nur die stimulierenden und regenerierenden Eigenschaften des Naphthalans, sondern wirken auch als Konservierungsmittel für den Komplex. In den Komplexen ''Vitavol'' und "Vitasu-1" werden die niedermolekularen Alkohole Glycerin, Sorbit, Propylenglycol, Dipropylenglycol eingesetzt, die als weichmachende und feuchtigkeitsspendende Bestandteile dienen und nicht nur die feuchtigkeitsspendenden Eigenschaften des vulkanischen Tons oder des jod- und bromhaltigen Wassers des Komplexes verstärken, sondern auch die Zeitspanne, während der die Hautmembran für die im vulkanischen Ton und im jod- und bromhaltigen Wasser enthaltenen Mineralsalze durchlässig ist, verlängern.

Die kosmetischen Mittel und Mittel für die Gesundheitspflege, die auf der Basis der erfindungsgemäßen Biokomplexe hergestellt werden, stimulieren die Stoffwechselprozesse in den Hautgeweben, verbessern ihre regenerativen Eigenschaften, haben entzündungshemmende und wundheilende Wirkung, werden gut von der Haut aufgenommen und verbessern so ihre Funktionen.

Somit trägt die Verwendung der erfindungsgemäßen biologischen Wirkkomplexe "Naphtovit", "Vitavol" und "Vitasu-1" bei der Herstellung kosmetischer Mittel und Mittel für die Gesundheitspflege zur Erhöhung des biologischen Wertes der hergestellten Produkte bei und erweitert so deren Sortiment.

Beispiele konkreter Ausführungsformen der Komplexe sind in Tabelle 1 angeführt, in der die erfindungsgemäßen Parameter in den Beispielen 2 bis 4 verwendet sind und die Beispiele 1 und 5 außerhalb des erfindungsgemäßen Bereichs liegen, wobei diese keine abschließende Aufzählung möglicher erfindungsgemäßer Ausführungsformen darstellen.

Tabelle der Beispiele konkreter Ausführungsformen der Komplexe

**Tabelle 1**

| Komplexe und Bestandteile | | Gehalt der Bestandteile in den Beispielen, Gew.-% | | | | |
|---|---|---|---|---|---|---|
| | | 1 | 2 | 3 | 4 | 5 |
| "Naphtovit" | | | | | | |
| 1. Naphthalan | | 50,0 | 46,2 | 47,8 | 49,3 | 46,0 |
| 2. Vitamine: | | | | | | |
| | C | 8,0 | 8,1 | 8,0 | 7,9 | 8,0 |
| | E | 6,0 | 6,0 | 6,0 | 5,9 | 6,0 |
| | F | 6,0 | 6,1 | 6,0 | 5,9 | 6,0 |
| 3. Extrakte: | | | | | | |
| | der Rose | 0,7 | 0,6 | 0,7 | 0,6 | 0,6 |
| | der Geranie | 1,5 | 1,6 | 1,5 | 1,4 | 1,5 |
| | des Basilikums | 2,0 | 1,9 | 2,0 | 2,0 | 1,9 |
| 4. Wachs oder Lanolin | | 25,8 | 29,5 | 28,0 | 27,0 | 30,0 |
| | | | | | | |

| "Vitavol" | | | | | | |
|---|---|---|---|---|---|---|
| 1. Ton | | 56,0 | 55,6 | 55,0 | 54,4 | 54,0 |
| 2. Talkum oder Kaolin | | 18,0 | 17,6 | 18,0 | 18,4 | 19,0 |
| 3. Alkohol | | 26,0 | 26,8 | 27,0 | 27,2 | 27,0 |
| | | | | | | |

| "Vitasu-1" | | | | | | |
|---|---|---|---|---|---|---|
| 1. Jod- und bromhaltiges Wasser | | 89,5 | 89,4 | 78,5 | 67,0 | 67,0 |
| 2. Guarkernmehl | | 1,5 | 0,5 | 1,0 | 2,0 | 1,5 |
| 3. Trilon B | | 1,0 | 0,1 | 0,5 | 1,0 | 0,5 |
| 4. Alkohol | | 8,0 | 10,0 | 20,0 | 30,0 | 31,0 |

In den erfindungsgemäßen biologischen Komplexen sind die Mengen an BWS, den komplexbildenden Bestandteilen und den anderen Komponenten so gewählt, dass sich nicht nur eine synergistische Wirkung ergibt, wodurch das kosmetische und biologische Potential des Produkts gesteigert wird, sondern die Komplexe auch in stabiler Form erhalten werden. So werden beispielsweise der Synergismus des Komplexes und dessen biologische Verträglichkeit gestört, wenn der BWS in einer Menge genommen wird, die unterhalb oder oberhalb des angegebenen Bereichs liegt. Wenn die strukturbildenden Bestandteile in einer Menge genommen werden, die unterhalb oder oberhalb des angegebenen Bereichs liegt, werden die Struktur und die Funktionen des Komplexes gestört.

Die erfindungsgemäßen Komplexe können mit üblichen Methoden hergestellt werden. Nachstehend sind einige konkrete Herstellungsbeispiele angegeben.

### Beispiel 1. Herstellung des biologischen Wirkkomplexes "Naphtovit"

Ein Reaktor wird mit 28 kg Bienenwachs oder Lanolin befüllt, dann werden die Vitamine E, C und F in einer Menge von 6,8 bzw. 6 kg hinzugegeben und auf 70°C erwärmt. Darauf wird der Mischer mit 40 - 50 U/min. eingeschaltet, und die erhaltene Mischung wird auf 85°C bis zur völligen Lösung der Bestandteile erwärmt. Die Mischung wird 30 Minuten lang gerührt, auf 60 - 65°C abgekühlt und das Naphthalan zugegeben. Weiter wird unter Rühren der Dispergator eingeschaltet, und die Mischung wird auf 40 - 45°C abgekühlt. Darauf wird der Dispergator abgeschaltet, die Mischung wird unter Rühren auf 30°C abgekühlt, und es werden die ätherischen Öle der Geranie, der Rose und des Basilikums in einer Menge von 1,5, 0,7 bzw. 2,0 kg zugegeben. Der Dispergator wird für 20 - 30 Minuten eingeschaltet, die erhaltene Mischung auf 25°C abgekühlt, 6 - 12 Stunden bei Raumtemperatur stehen gelassen und abgefüllt.

Der erhaltene Komplex liegt als dicke, ölige, gelbliche Flüssigkeit mit dem für diese Mischung typischen Geruch vor und enthält keine toxischen Elemente, Schwermetalle, Pilze oder Mikroben. Der pH-Wert beträgt 6,00.

### Beispiel 2. Herstellung des biologischen Wirkkomplexes "Vitavol"

Ein Reaktor wird mit 55 kg vulkanischen Tons befüllt. Unter Rühren werden 27 kg Glycerin oder Dipropylenglycol langsam zugegeben. Das Gemisch wird mit einer Dispergatorleistung von 3000 - 6000 U/min. 20 - 30 Minuten lang intensiv dispergiert. Danach werden unter langsamem Rühren 18 kg Talkum oder Kaolin zugegeben und weitere 20 - 30 Minuten gerührt sowie zusätzlich 10 - 20 Minuten dispergiert. Danach wird das Gemisch in Behälter zur Lagerung abgefüllt.

Der erhaltene Komplex ist eine dicke, homogene, pastöse, hellgraue, geruchlose Masse ohne mechanische Verunreinigungen, toxische Elemente, Schwermetalle, Pilze oder Mikroben. Der pH-Wert beträgt 8,93 (1 %-ige wässrige Lösung).

### Beispiel 3. Herstellung des biologischen Wirkkomplexes "Vitasu-1"

Ein Reaktor wird mit 20 kg eines der niedermolekularen Alkohole oder ihrer Mischung befüllt. Unter Rühren werden 1,0 kg Guarkernmehl und nach dessen vollständiger Auflösung 0,5 kg Trilon B zugegeben. Das Gemisch wird bis zur Bildung einer klaren Lösung gerührt und dann in Behälter zur Lagerung abgefüllt.

Der erhaltene Komplex stellt eine durchsichtige, leicht opake, ölige, geruchlose und farblose Flüssigkeit mit einem pH-Wert von 6,37 dar. Der Komplex ist ungiftig und enthält keine Schwermetalle.

Die Komplexe "Naphtovit", "Vitavol" und "Vitasu-1" werden in Rezepturen für kosmetische Erzeugnisse und Erzeugnisse für die Gesundheitspflege verwendet. Bevorzugt werden die erfindungsgemäßen Komplexe am Ende der Herstellung der Rezeptur beigemischt. Tabelle 2 enthält eine Aufzählung von kosmetischen Mitteln und Mitteln für die Gesundheitspflege, in denen die erfindungsgemäßen Komplexe verwendet werden können. Die verwendete Menge an erfindungsgemäßem Komplex ist ebenfalls angegeben.

**Tabelle 2.**

| Kosmetische Mittel und Mittel für die Gesundheitspflege | | Biologische Wirkkomplexe, % | | |
|---|---|---|---|---|
| | | Naphtavit | Vitavol | Vitasu-1 |
| 1 | Cremes für Gesicht, Körper, Hände, Füße | 2,0-10,0 | 2,0-10,0 | 0,5-5,0 |
| 2 | Augencremes | 0,2-1,0 | 0,2-1,0 | 0,2-1,0 |
| 3 | Gesichts- und Körpermasken | 10,-30,0 | 20,0-60,0 | 10,0-30,0 |
| 4 | Gels für Gesicht, Hände, Füße, Körper | 0,05-1,5 | 0,05-1,5 | 0,05-1,5 |
| 5 | Flüssige Creme für Gesicht, Hände, Füße, Körper | 1,0-5,0 | 2,0-8,0 | 0,1-2,0 |
| 6 | Alkoholische und leicht alkoholische Lotionen | 1,0-5,0 | 1,0 - 2,0 | 1,0-2,0 |
| 7 | Nicht alkoholische Lotionen und Tonika | 0,05-0,5 | 0,05-0,5 | 0,5-1,0 |
| 8 | Shampoos, Duschgels, Badeschäume | 0,5-5,0 | 0,5-5,0 | 0,5-5,0 |
| 9 | Haarbalsame, Haarsprays, Haarpackungen, Haarspülungen | 0,5-8,0 | 4,0-12,0 | 5,0-10,0 |
| 10 | Flüssige und cremeförmige dekorative Kosmetik | 0,5-1,5 | 1,0-10,0 | 0,5-1,5 |

## Patentansprüche

1. Biologischer Wirkkomplex zur Herstellung von Parfümerie- und kosmetischen Erzeugnissen, enthaltend einen biologischen Wirkstoff (BWS) und komplexbildende Bestandteile, ***dadurch gekennzeichnet, dass*** er als BWS Naphthalan und als komplexbildende Bestandteile die Vitamine C, E und F, ätherische Öle der Rose, der Geranie und des Basilikums sowie strukturbildende Bestandteile enthält, bei folgendem Verhältnis der Komponenten (Gew.-%):
| | | |
|---|---|---|
| Naphtalan | | 49,3 - 46,2 |
| Vitamine | | |
| | C | 7,9 - 8,1 |
| | E | 5,9 - 6,1 |
| | F | 5,9 - 6,1 |
| ätherische Öle | | |
| | der Rose | 0,6 - 0,7 |
| | der Geranie | 14,0 - 16,0 |
| | des Basilikums | 19,0 - 21,0 |
| strukturbildende | | |
| | Bestandteile | 26,0 - 30,0. |

2. Biologischer Wirkkomplex nach Anspruch 1, ***dadurch gekennzeichnet, dass*** die strukturbildenden Bestandteile Bienenwachs und/oder Lanolin umfassen.

3. Biologischer Wirkkomplex zur Herstellung von Parfümerie- und kosmetischen Erzeugnissen, enthaltend einen biologischen Wirkstoff (BWS) und komplexbildende Bestandteile, ***dadurch gekennzeichnet, dass*** er als BWS vulkanischen Ton und als komplexbildenden Bestandteil einen Füllstoff sowie niedermolekulare Alkohole enthält, bei folgendem Verhältnis der Komponenten (Gew.-%):
| | |
|---|---|
| vulkanischer Ton | 55,6 - 54,4 |
| Füllstoff | 17,6 - 18,4 |
| niedermolekulare Alkohole | 26,8 - 27,2. |

4. Biologischer Wirkkomplex nach Anspruch 3, ***dadurch gekennzeichnet, dass*** der Füllstoff Talkum und/oder Kaolin umfaßt.

5. Biologischer Wirkkomplex nach Anspruch 3, ***dadurch gekennzeichnet, dass*** die niedermolekularen Alkohole Glycerin und/oder Propylenglycol darstellen.

6. Biologischer Wirkkomplex zur Herstellung von Parfümerie- und kosmetischen Erzeugnissen, enthaltend einen biologischen Wirkstoff (BWS) und komplexbildende Bestandteile, ***dadurch gekennzeichnet, dass*** er als BWS jod- und bromhaltiges Wasser und als komplexbildende Bestandteile Guarkemmehl und Trilon B sowie niedermolekulare Alkohole enthält, bei folgendem Verhältnis der Komponenten (Gew.-%):
| | |
|---|---|
| jod- und bromhaltiges Wasser | 89,4 - 67,0 |
| Guarkernmehl | 0,5 - 2,0 |
| Trilon B | 0,1 - 1,0 |
| niedermolekulare Alkohole | 10,0 - 30,0. |

7. Biologischer Wirkkomplex nach Anspruch 6, ***dadurch gekennzeichnet, dass*** die niedermolekularen Alkohole Glycerin und/oder Sorbit und/oder Propylenglycol und/oder Dipropylenglycol darstellen.
